Europäisches Patentamt

European Patent Office

Office européen des brevets

(19)

(11) Numéro de publication: **0 192 574**
**B1**

(12)

# FASCICULE DE BREVET EUROPEEN

(45) Date de publication du fascicule du brevet:
**31.05.89**

(51) Int. Cl.⁴: **A61M 1/10**

(21) Numéro de dépôt: **86400345.4**

(22) Date de dépôt: **19.02.86**

(54) Pompe d'assistance circulatoire et coronaire à ballonnets intra-aortiques.

(30) Priorité: **20.02.85 FR 8502428**

(43) Date de publication de la demande:
**27.08.86 Bulletin 86/35**

(45) Mention de la délivrance du brevet:
**31.05.89 Bulletin 89/22**

(84) Etats contractants désignés:
**AT BE CH DE FR GB IT LI LU NL SE**

(56) Documents cités:
**FR-A- 2 361 123**
**US-A- 3 585 983**
**US-A- 3 692 018**
**US-A- 4 014 317**
**US-A- 4 244 362**

(73) Titulaire: **MEDICORP RESEARCH LABORATORIES CORPORATION, 1200 North Federal Highway Suite 200-26, Boca Raton Florida 33432(US)**

(72) Inventeur: **Karcher, Gilles, 2, Rue Lafayette, F-54000 Nancy(FR)**
Inventeur: **Amor, Max, 9, Square de Liège, F-54500 Vandoeuvre(FR)**
Inventeur: **Niddam, Roger, 43, Allée du Jardin Anglais, F-93340 Le Rancy(FR)**
Inventeur: **Villemot, Jean-Pierre, Le Trident Rue Cyffle, F-54000 Nancy(FR)**

(74) Mandataire: **Laget, Jean-Loup et al, Cabinet Pierre Loyer 77, rue Boissière, F-75116 Paris(FR)**

## Description

L'invention concerne une pompe d'assistance circulatoire et coronaire à ballonnets intra-aortiques.

Dans certaines situations d'insuffisance cardiaque pré- ou postopératoires, il est indispensable d'aider la fonction cardiaque pour assurer notamment une meilleure circulation coronaire. A cet effet, il est connu d'utiliser le système dit de contre-pulsion par ballonnet intra-aortique. Un ballonnet, placé dans l'aorte descendante est gonflé et dégonflé, sous le contrôle de l'électrocardiagraphe (ECG), de façon à augmenter la pression diastolique intra-aortique, et par voie de conséquence la pression de perfusion coronaire.

Cependant, le ballonnet étant placé loin du cœur et, en particulier, en aval des tronces supra-aortiques, il a une faible influence sur la situation coronaire, qui peut elle-même être à l'origine de la faiblesse du débit cardiaque. Par ailleurs, ce ballonnet est d'un volume important, et il nécessite une sonde de fort diamètre, donc rigide et encombrante. Enfin, la commande du ballonnet en fonction du rythme cardiaque présente des lacunes.

Un dispositif d'assistance cardiaque intra-artérielle est décrit par le document US-A 3 585 983. Ce dispositif comporte un ballon non élastique qui est gonflé périodiquement pour augmenter la diastole. L'extrémité du dispositif, qui est introduit dans l'aorte comme un catheter, comporte un capteur de pression. Selon ce document, le ballon est placé juste en dessous de l'embranchement des artères sous-clavières, c'est-à-dire assez loin du cœur et donc des artères coronaires.

Un premier but de l'invention est de proposer une pompe d'assistance coronaire qui agisse au plus près des artères coronaires en imposant à l'organisme un traumatisme aussi minime que possible.

Un autre but de l'invention est de prévoir une commande de ballonnet qui soit affranchie des parasites auxquels est sujet l'électrocardiographe.

La présente invention a pour objet une pompe d'assistance circulatoire et coronaire qui comprend:

– une sonde aboutissant à un ballonnet intra-aortique de pulsion;

– une pompe assurant le gonflage et le dégonflage du ballonnet;

– un capteur de pression monté à l'extrémité de la sonde et destiné à mesurer la pression régnant dans l'aorte en regard des valves cardiaques;

– un circuit électronique qui assure le gonflage et le dégonflage du ballonnet pendant la diastole en prenant en compte à la fois les signaux d'un électrocardiographe (ECG) et les signaux du capteur de pression, caractérisée en ce que la pompe comprend de plus:

– une sonde aboutissant à un ballonnet d'obturation de l'aorte et relié à la pompe de gonflage et de dégonflage,

– un guide dont l'extrémité est destinée à être fixée dans la pointe du ventricule gouche pour assurer l'arrimage des sondes et des ballonnets;

– et en ce que le circuit électronique assure de plus le gonflage et le dégonflage du ballonnet d'obturation en séquence avec le gonflage et le dégonflage du ballonnet de pulsion.

Selon d'autres caractéristiques de l'invention:

- le premier ballonnet est placé dans l'aorte ascendante en amont des tronces supra-aortiques, et le second ballonnet en amont du premier ballonnet, en regard des artères coronaires, de sorte que le gonflage du premier ballonnet assure l'obturation de l'aorte et celui du second ballonnet une pulsion vers les artères coronaires d'un volume de sang sensiblement égal au volume du second ballonnet,

- le circuit électronique comprend pour la commande de la pompe, un micro-processeur recevant: une mesure du temps écoulé depuis la dernière onde R de l'ECG, une mesure numérique de la pression instantanée dans l'aorte au droit du capteur, et une mesure du potentiel endo-cavitaire,

- la mesure de la pression instantanée est obtenue au moyen d'un convertisseur analogique/numérique dont la fréquence d'échantillonnage est de l'ordre de 100 à 500 par seconde,

- la mesure du temps écoulé depuis la dernière onde R de l'ECG est obtenue au moyen d'un compteur qui compte les impulsions d'une horloge et qui est remis à zéro par une impulsion synchrone de l'onde R de l'ECG,

- l'impulsion synchrone de l'onde R de l'ECG est l'impulsion de sortie d'un comparateur qui reçoit sur son entrée - un signal correspondant à la somme des signaux des trois premières dérivations habituelles d'un ECG, et sur son entrée + la valeur moyenne dudit signal obtenue par un intégrateur.

- le guide assure la mesure du potentiel endo-cavitaire, et éventuellement une stimulation du coeur.

D'autres caractéristiques ressortent de la description qui suit faite avec référence au dessin annexé sur lequel on peut voir:

Figure 1, un schéma simplifié du principe de réalisation de la pompe d'assistance circulatoire et coronaire selon l'invention;

Figure 2, un schéma symbolique simplifié d'un mode de réalisation du circuit électronique de commande de la pompe de la figure 1.

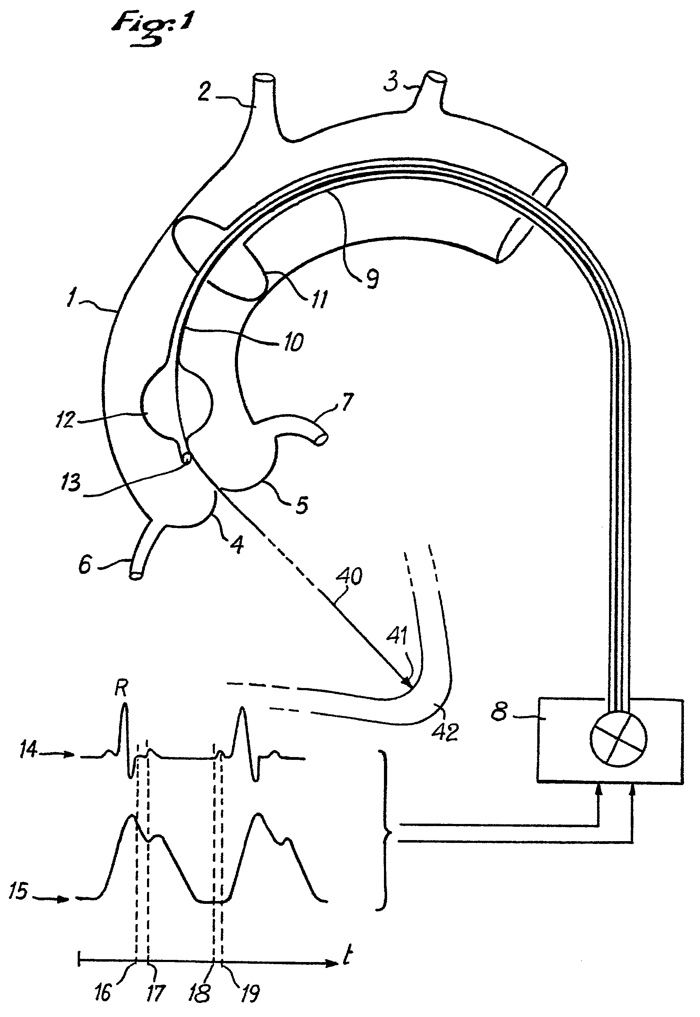

En se reportant à la figure 1, on peut voir une aorte 1 et l'amorce des tronces supra-aortiques droit 2 et gauche 3. La masse cardiaque n'est pas représentée et elle est symbolisée par les valves cardiaques 4, 5 et les artères coronaires droite 6 et gauche 7.

La pompe d'assistance est schématisée en 8. Elle est susceptible d'alimenter, en gaz par exemple, deux sondes 9 et 10, qui sont représentées coaxiales mais peuvent être disposées de toute manière appropriée. Chacune de ces deux sondes aboutit à un ballonnet gonflable : la sonde 9 au premier ballonnet 11, ou ballonnet d'obturation ; la sonde 10 au second ballonnet 12, ou ballonnet de pulsion. Enfin, la sonde 10 porte à son extrémité, au-delà du ballonnet 12, un capteur de pression 13 situé juste en regard des valves cardiaques 4 et 5.

Un guide 40 est encore prévu pour assurer une certaine rigidité aux sondes 9 et 10. Ce guide se termine par une extrémité 41 qui est fixée dans la pointe du ventricule gauche, symbolisée en 42. La fixation doit être prévue rétractable car la pompe d'assistance est mise en place de façon temporaire. Le guide 40 a trois fonctions. Tout d'abord il assure l'arrimage des ballonnets et des sondes dans l'aorte ascendante. Il évite ainsi le déplacement des ballonnets sous l'effet du flux sanguin systolique. Ensuite, il assure une détection du potentiel endocavitaire, ce qui permet de s'affranchir des parasites et des troubles de la conduction pour commander la synchronisation des opérations d'assistance cardiaque. Enfin, il peut jouer le rôle habituel d'un stimulateur cardiaque, en cas de besoin.

Le ballonnet 11 d'obturation est placé en amont des artères carotides, dans l'aorte ascendante. Lorsqu'il est gonflé, il obture l'aorte et isole en amont une cavité qui s'étend jusqu'aux valves 4, 5. Le ballonnet 12 de pulsion est placé en amont du ballonnet 11, dans l'aorte ascendante, et en regard des artères coronaires 6 et 7.

Le fonctionnnement de la pompe d'assistance est le suivant, et il intervient pendant la diastole : la pompe 8 gonfle le ballonnet 11 par l'intermédiaire de la sonde 9, jusqu'à obturer l'aorte ; elle gonfle ensuite le ballonnet 12 par l'intermédiaire de la sonde 10, ce qui permet d'envoyer dans les artères coronaires 6 et 7 un volume de sang imposé et correspondant sensiblement à celui du ballonnet 12. Ensuite les ballonnets sont dégonflés avant la systole, et le cycle recommence à la diastole suivante.

Sur la figure 1 sont schématisés les signaux représentatifs, en fonction du temps t, d'un électrocardiogramme 14 et de la pression 15 régnant dans l'aorte au niveau du capteur de pression 13. Sur l'échelle des temps, on voit que le gonflage du premier ballonnet 11 intervient au point 16, celui du deuxième ballonnet 12 intervient au point 17, tandis que le dégonflage du premier ballonnet 11 intervient au point 18 et celui du deuxième ballonnet 12 au point 19.

Le gonflage sélectif des ballonnets doit être déclenché en début de diastole, et le dégonflage en fin de diastole, de façon à ne pas gêner la circulation sanguine dans l'aorte. La séquence de gonflage des ballonnets et la synchronisation correspondante des opérations est très importante pour l'efficacité hémodynamique de ce système d'assistance circulatoire. C'est pourquoi cette synchronisation est assurée à la fois sur les signaux d'un ECG et sur les signaux de pression fournis par le capteur 13.

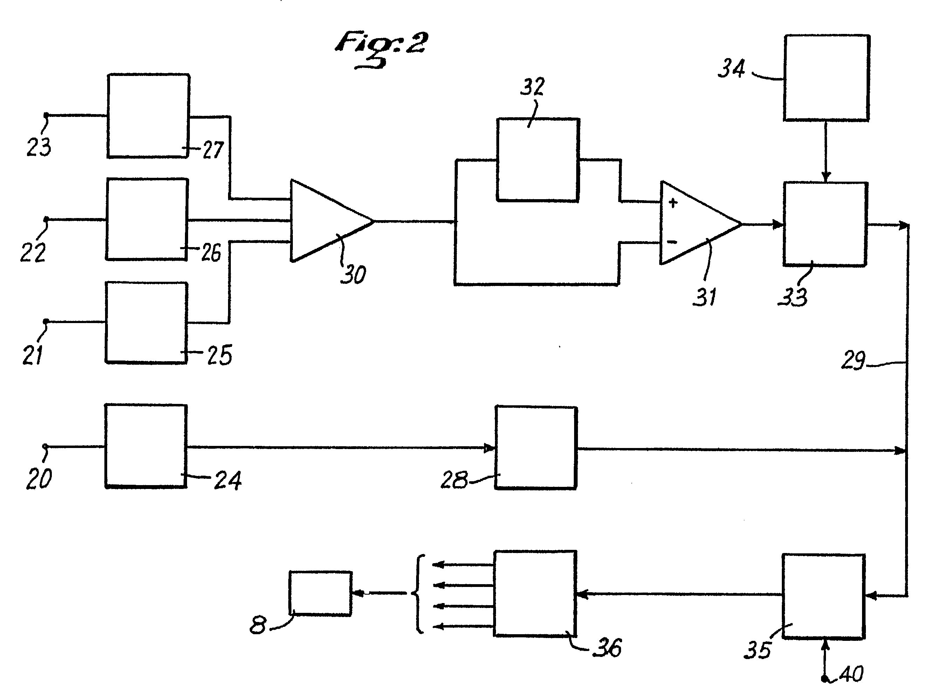

Le circuit électronique de commande de la pompe 8 est représenté figure 2. Le signal de pression arrive en 20, et les signaux des trois premières dérivations classiques d'un électrocardiographe, ECG 1, ECG 2, ECG 3, arrivent respectivement en 21, 22, 23.

Les quatre signaux 20 à 23 sont soumis à un filtrage dans les filtres passe-bas 24 à 27. Le signal de pression est ensuite soumis à une conversion analogique/numérique dans un convertisseur 28 dont la fréquence d'échantillonnage est de l'ordre de 100 à 500 par seconde. Le signal numérique correspondant est appliqué à un bus 29.

Les trois signaux d'ECG sont traités de façon que les ondes R soient dans le sens positif et, à la sortie des filtres 25 à 27 ils sont appliqués à un sommateur 30. Le signal de sortie du sommateur 30 est appliqué d'une part, directement à l'entrée - d'un comparateur 31, et d'autre part, par l'intermédiaire d'un intégrateur 32 qui en donne la valeur moyenne, à l'entrée + du comparateur 32. Ainsi, le comparateur compare en permanence la valeur instantanée du signal à sa valeur moyenne, et il ne délivre une impulsion de sortie que pendant l'intervalle de temps où l'onde R dépasse la valeur moyenne du signal. Cette impulsion de sortie du comparateur 31 est appliquée à l'entrée de remise à zéro d'un compteur 33. Ce compteur compte en permanence les impulsions en provenance d'une horloge 34 et il transmet au bus 29 des nombres correspondant au temps écoulé depuis la dernière onde R qui a remis le compteur à zéro. Le bus 29 transmet donc à un microprocesseur 35 d'une part une mesure du temps écoulé depuis la dernière onde R de l'ECG, d'autre part une mesure numérique de la pression instantanée dans l'aorte au droit du capteur 13.

Le micro-processeur 35 reçoit par ailleurs du guide 40 une mesure du potentiel endo-cavitaire, et il commande par l'intermédiaire d'un module de sortie 36 les ouvertures et fermetures de la pompe 8 auxquelles correspondent les points 16 à 19 de gonflage et de dégonflage des ballonnets 11 et 12.

Par la prise en compte des signaux d'ECG, des signaux de pression dans l'aorte, et du potentiel endocavitaire, le circuit électronique de commande de la pompe 8 permet de s'affranchir des parasites auxquels sont sujets les ECG. L'assistance circulatoire et coronaire assurée par la pompe selon l'invention se révèle, en conséquence, particulièrement efficace.

**Revendications**

1. Pompe d'assistance circulatoire et coronaire qui comprend:
   - une sonde (10) aboutissant à un ballonnet (12) intra-aortique de pulsion;
   - une pompe (8) assurant le gonflage et le dégonflage du ballonnet (12);
   - un capteur (13) de pression monté à l'extrémité de la sonde (10) et destiné à mesurer la pression règnant dans l'aorte en regard des valves cardiaques;
   - un circuit électronique qui assure le gonflage et le dégonflage du ballonnet (12) pendant la diastole en prenant en compte à la fois les signaux d'un électrocardiographe (ECG) et les signaux du capteur (13) de pression, caractérisée en ce que la pompe comprend de plus:
   - une sonde (9) aboutissant à un ballonnet (11) d'obturation de l'aorte et relié à la pompe (8) de gonflage et de dégonflage,
   - un guide (40) dont l'extrémité est destinée à être fixée dans la pointe du ventricule gauche (42) pour assurer l'arrimage des sondes (9, 10) et des ballonnets (11, 12);
   - et en ce que le circuit électronique assure de plus le gonflage et le dégonflage du ballonnet (9) d'obturation en séquence avec le gonflage et le dégonflage du ballonnet (12) de pulsion.

2. Pompe selon la revendication 1, caractérisée en ce que le circuit électronique comprend pour la commande de la pompe (8) un micro-processeur (35) recevant une mesure du temps écoulé depuis la dernière onde R de l'ECG, une mesure numérique de la pression instantanée dans l'aorte au droit du capteur (13), et une mesure du potentiel endo-cavitaire.

3. Pompe selon la revendication 2, caractérisée en ce que la mesure de la pression instantanée est obtenue au moyen d'un convertisseur analogique/numérique dont la fréquence d'échantillonnage est de l'ordre de 100 à 500 par seconde.

4. Pompe selon la revendication 2, caractérisée en ce que la mesure du temps écoulé depuis la dernière onde R de l'ECG est obtenue au moyen d'un compteur (33) qui compte les impulsions d'une horloge (34) et qui est remis à zéro par une impulsion synchrone de l'onde R de l'ECG.

5. Pompe selon la revendication 4, caractérisée en ce que l'impulsion synchrone de l'onde R de l'ECG est l'impulsion de sortie d'un comparateur (31) qui reçoit sur son entrée − un signal correspondant à la somme des signaux des trois premières dérivations habituelles d'un ECG, et sur son entrée + la valeur moyenne dudit signal obtenue par un intégrateur (32).

6. Pompe selon la revendication 2, caractérisée en ce que le guide (40) assure la mesure du potentiel endo-cavitaire, et éventuellement une stimulation du cœur.

**Patentansprüche**

1. Pumpe zur Unterstützung des Blutkreislaufs und der Herzkranzgefäße, welche Pumpe umfaßt:
   − eine zu einem in der Aorta angeordneten Pulsationsballon (12) führende Sonde (10);
   − eine Pumpe (8) zum Aufblasen und Entleeren des Ballons (12);
   − einen am Ende der Sonde (10) angeordneten Druckfühler (13), der zum Messen des in der Aorta gegenüber den Herzklappen herrschenden Drucks dient;
   − eine elektronische Schaltung, die das Aufblasen und Entleeren des Ballons (12) während der Diastole sicherstellt, indem sie zugleich die Signale des Elektrokardiographen (EKG) und die Signale des Druckfühlers (13) übernimmt, dadurch gekennzeichnet, daß die Pumpe außerdem aufweist:
   − eine Sonde (9), die zu einem die Aorta verschließenden Ballon (11) führt, der an eine Aufblas- und Entleerungspumpe (8) angeschlossen ist,
   − ein Führungselement (40), dessen Ende in der Spitze der linken Herzkammer (42) befestigt werden soll, um die Festlegung der Sonden (9, 10) und der Ballone (11, 12) sicherzustellen;
   − und dadurch, daß die elektronische Schaltung außerdem das Aufblasen und Entleeren des Verschließballons (11) in Abfolge mit dem Aufblasen und Entleeren des Pulsationsballons (12) sicherstellt.

2. Pumpe nach Anspruch 1, dadurch gekennzeichnet, daß die elektronische Schaltung für die Steuerung der Pumpe (8) einen Mikroprozessor (35) aufweist, der einen Meßwert für die Zeit, die seit der letzten R-Welle des EKG verstrichen ist, einen digitalen Meßwert für den Augenblicksdruck in der Aorta am Ort des Druckfühlers (13) und einen Meßwert für das endokavitäre Potential empfängt.

3. Pumpe nach Anspruch 2, dadurch gekennzeichnet, daß der Meßwert für den Augenblicksdruck gewonnen wird mit Hilfe eines Analog/Digital-Umsetzers, dessen Abtastfrequenz größenordnungsmäßig 100 bis 500 Hz beträgt.

4. Pumpe nach Anspruch 2, dadurch gekennzeichnet, daß der Meßwert für die seit der letzten R-Welle des EKG verstrichene Zeit gewonnen wird mit Hilfe eines Zählers (33), der die Impulse eines Impulsgebers (34) zählt und der auf Null gestellt wird durch einen mit der R-Welle des EKG synchronen Impuls.

5. Pumpe nach Anspruch 4, dadurch gekennzeichnet, das der mit der R-Welle des EKG synchrone Impuls der Ausgangsimpuls eines Komparators (31) ist, der an seinem −Eingang ein Signal empfängt, das der Summe der drei ersten üblichen Ableitungen eines EKG entspricht, und an seinem +Eingang den Mittelwert des genannten Signals, das von einem Integrierglied (32) gewonnen wird.

6. Pumpe nach Anspruch 2, dadurch gekennzeichnet, daß das Führungselement (40) die Messung des endokavitären Potentials und gegebenenfalls die Stimulierung des Herzens ermöglicht.

**Claims**

1. A circulatory and coronary assistance pump comprising:
   − a catheter (10) ending in an intra-aortic pulsion balloon (12);
   − a pump (8) to inflate and deflate the balloon (12);
   − a pressure sensor (13) mounted on the end of the catheter (10) to measure the pressure within the aorta opposite the heart valves;
   − an electronic circuit which inflates and deflates the balloon (12) during the diastole, taking into account both the signals from an electrocardiograph (ECG) and the signals from the pressure sensor (13); characterised in that the pump comprises in addition:
   − a catheter (9) ending in an aorta-closure balloon (11) and connected to the inflating and deflating pump (8),
   − a guide (40), the end of which is to be fixed into the apex of the left ventricle (42) to secure the catheters (9, 10) and the balloons (11, 12);
   − and in that the electronic circuit alo inflates and deflates the closure balloon (9) in sequence with the inflation and deflation of the pulsion balloon (12).

2. A pump according to Claim 1, characterised in that in order to control the pump (8) the electronic circuit comprises a micro-processor (35) which receives a measurement of the time which has lapsed since the previous wave R of the ECG, a digital measurement of the instantaneous pressure within the aorta to the right of the sensor (13), and a measurement of the endo-cavitary potential.

3. A pump according to Claim 2, characterised in that the measurement of the instantaneous pressure is obtained by means of an analogue-digital converter, the sampling frequency of which is of the order of 100 to 500 per second.

4. A pump according to Claim 2, characterised in that the measurement of time which has lapsed since the previous wave R of the ECG is obtained by means of a counter (33) which counts the pulses of a clock (34) and which is reset at zero by a synchronous pulse of the wave R of the ECG.

5. A pump according to Claim 4, characterised in that the synchronous pulse of the wave R of the ECG is the output pulse of a comparator (31) which at its negative input receives a signal corresponding to the total of the signals of the first three habitual derivations of an ECG, and at its positive input the mean value of said signal obtained by an integrator (32).

6. A pump according to Claim 2, characterised in that the guide (40) measures the endo-cavitary potential and if necessary stimulates the heart.

Fig:1

Fig:2

EP 0 192 574 B1